(19) Europäisches Patentamt
European Patent Office
Office européen des brevets

(11) Veröffentlichungsnummer: **0 449 049 B1**

# (12) EUROPÄISCHE PATENTSCHRIFT

(45) Veröffentlichungstag der Patentschrift: **28.12.94**

(51) Int. Cl.$^5$: **C07C 69/76**, C07C 67/08, C09K 19/32, C09K 19/46, G02F 1/137

(21) Anmeldenummer: **91103983.2**

(22) Anmeldetag: **15.03.91**

## (54) Naphthalindicarbonsäureester.

(30) Priorität: **27.03.90 CH 1009/90**

(43) Veröffentlichungstag der Anmeldung:
**02.10.91 Patentblatt 91/40**

(45) Bekanntmachung des Hinweises auf die Patenterteilung:
**28.12.94 Patentblatt 94/52**

(84) Benannte Vertragsstaaten:
**CH DE FR GB IT LI NL**

(56) Entgegenhaltungen:
**DE-A- 3 534 778**
**GB-A- 1 603 075**

**PATENT ABSTRACTS OF JAPAN, unexamined applications, Sektion C, Band 13, Nr.315, 18. Juli 1989 THE PATENT OFFICE JAPANESE GOVERNMENT Seite 116 C 619**

(73) Patentinhaber: **F. HOFFMANN-LA ROCHE AG**
**Postfach 3255**
**CH-4002 Basel (CH)**

(72) Erfinder: **Kelly, Stephen, Dr.**
**Frankenstrasse 10**
**CH-4313 Möhlin (CH)**

(74) Vertreter: **Cottong, Norbert A. et al**
**Grenzacherstrasse 124**
**Postfach 3255**
**CH-4002 Basel (CH)**

EP 0 449 049 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue chirale Dotierstoffe für Flüssigkristalle und deren Herstellung sowie flüssigkristalline Gemische, die solche Dotierstoffe enthalten, und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise wird zur Verwendung in Anzeigevorrichtungen mit verdrillt nematischer Struktur bevorzugt ein nematischer Flüssigkristall mit optisch aktivem Zusatzstoff dotiert, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen auf der Basis von chiral getilteten smektischen Phasen können vorzugsweise aus einem Material mit getilteter smektischer Phase und einem oder mehreren optisch aktiven Dotierstoffen bestehen.

Die elektro-optischen Kennlinien von Flüssigkristall-Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von chiralem Dotierstoff, welcher eine mit steigender Temperatur abnehmende Ganghöhe induziert. Eine solche inverse Temperaturabhängigkeit wurde nur für wenige Verbindungen gefunden. Sie kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (DE-A-2827471). Allerdings erfordert dies meist einen relativ hohen Anteil an chiralen Dotierstoffen.

Cholesterische Flüssigkristalle reflektieren Licht in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner wurden cholesterische Flüssigkristalle auch verschiedentlich für thermochrome Anwendungen und in kosmetischen Präparaten verwendet.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasetyp des Flüssigkristallmaterials gut kompatibel sein und den Mesophasenbereich nicht zu stark einschränken. Solche Eigenschaften wären auch für chirale Dotierstoffe zur Erzielung der eingangs genannten, verdrillt nematischen Strukturen erwünscht, da ihr Anteil so niedrig gehalten werden könnte, dass die Eigenschaften des Flüssigkristallmaterials nur unwesentlich beeinflusst werden.

Die Erfindung betrifft optisch aktive Naphthalin-2,6-dicarbonsäureester der allgemeinen Formel

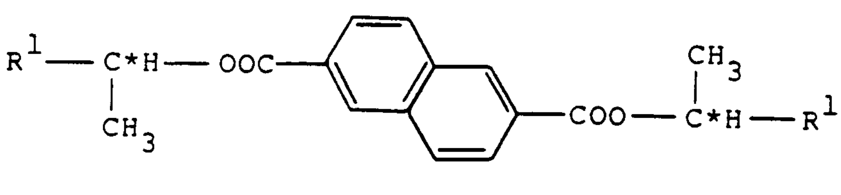

I

worin C* ein chirales Kohlenstoffatom bezeichnet und $R^1$ $C_2$-$C_{12}$-Alkyl, $C_2$-$C_{12}$-Alkenyl, $C_2$-$C_{12}$-Alkoxycarbonyl oder $C_3$-$C_{12}$-Alkenyloxycarbonyl bedeutet.

Die Verbindungen der Formel I sind gut löslich in üblichen Flüssigkristallmaterialien und ermöglichen eine hohe Verdrillung der Flüssigkristallstruktur. Die Klärpunkte von Flüssigkristallen werden bei Zusatz von Verbindungen der Formel I in der Regel nicht oder nur unwesentlich gesenkt. Die Verbindungen der Formel I sind leicht herstellbar, relativ niederviskos und ausreichend stabil gegen elektrische und magnetische

Felder. Sie erfüllen daher in optimaler Weise die oben genannten Anforderungen.

Es versteht sich, dass zur Erzielung optischer Aktivität und eines hohen Verdrillungsvermögens beide in Formel I dargestellten chiralen Kohlenstoffatome R-Konfiguration oder beide S-Konfiguration aufweisen sollten.

$R^1$ kann verzweigt-kettig und gegebenenfalls chiral sein. Im allgemeinen sind jedoch geradkettige Reste $R^1$ bevorzugt.

Der obige Ausdruck "$C_2$-$C_{12}$-Alkyl" umfasst Reste wie Aethyl, Propyl, Butyl, Pentyl, Hexyl, Heptyl, Octyl, Nonyl, Decyl und dergleichen.

Der Ausdruck "$C_2$-$C_{12}$-Alkenyl" umfasst Reste wie Vinyl, Allyl, Propenyl, Butenyl, Pentenyl, Hexenyl, Heptenyl, Octenyl, Nonenyl, Decenyl und dergleichen.

Der Ausdruck "$C_2$-$C_{12}$-Alkoxycarbonyl" umfasst Reste wie Methoxycarbonyl, Aethoxycarbonyl, Propyloxycarbonyl, Butyloxycarbonyl, Pentyloxycarbonyl, Hexyloxycarbonyl, Heptyloxycarbonyl, Octyloxycarbonyl und dergleichen.

Der Ausdruck "$C_3$-$C_{12}$-Alkenyloxycarbonyl" umfasst Reste wie Vinyloxycarbonyl, Allyloxycarbonyl, Propenyloxycarbonyl, Butenyloxycarbonyl, Pentenyloxycarbonyl, Hexenyloxycarbonyl, Heptenyloxycarbonyl, Octenyloxycarbonyl, 2,6-Dimethyl-5-hepten-1-yloxycarbonyl und dergleichen. Bevorzugt sind im allgemeinen 2E-Alkenyloxycarbonylgruppen und Alkenyloxycarbonylgruppen mit endständiger Doppelbindung.

Besonders bevorzugt sind im allgemeinen diejenigen Verbindungen der Formel I, worin $R^1$ höchstens 7 Kohlenstoffatome aufweist.

Die Verbindungen der Formel I können erfindungsgemäss dadurch hergestellt werden, dass man Naphthalin-2,6-dicarbonsäure oder ein geeignetes Derivat der Naphthalin-2,6-dicarbonsäure mit einer optisch aktiven Verbindung der allgemeinen Formel

$$\begin{array}{c} CH_3 \\ | \\ HO\text{-}C^{*}H\text{-}R^1 \end{array} \qquad II$$

worin C* und $R^1$ die obigen Bedeutungen haben,
verestert.

Die Veresterung kann in an sich bekannter Weise erfolgen. Geeignete Derivate der Naphthalin-2,6-dicarbonsäure sind beispielsweise die Säurehalogenide, z.B. Naphthalin-2,6-dicarbonsäurechlorid. Eine bevorzugte Methode ist die Umsetzung eines Naphthalin-2,6-dicarbonsäurehalogenids in einem inerten organischen Lösungsmittel (z.B. einem aromatischen oder gesättigten, gegebenenfalls chlorierten Kohlenwasserstoff, wie Benzol, Toluol, Hexan oder Tetrachlorkohlenstoff) in Gegenwart eines säurebindenden Mittels (z.B. Pyridin).

Die Ausgangsmaterialien sind bekannte oder Analoge bekannter Verbindungen.

Die Erfindung betrifft ebenfalls ein flüssigkristallines Gemisch enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60 °C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew.% betragen. Für Anzeigevorrichtungen auf der Basis von Flüssigkristallen mit verdrillt nematischer Struktur ist je nach Zellentyp und -dicke meist eine Ganghöhe von etwa 3-40 µm erforderlich und daher ein entsprechend kleiner Anteil ausreichend (meist etwa 0,1-1 Gew.%). Demgegenüber werden für Anwendungen, welche auf der Reflexion von sichtbarem Licht durch cholesterische Schichten beruhen, Ganghöhen von weniger als 2 µm, beispielsweise etwa 0,4-0,6 µm benötigt, was einen entsprechend höheren Anteil an chiralem Dotierstoff erfordert (meist etwa 2-20 Gew.%). Für Zellen mit hochverdrillter nematischer Struktur beträgt der Anteil an chiralem Dotierstoff meist etwa 0,3-3 Gew.%.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien

bevorzugt. Grundsätzlich kann aber auch eine flüssigkristalline Verbindung als Trägermaterial verwendet werden, wenn sie eine ausreichend breite Mesophase aufweist.

Als Komponenten für flüssigkristalline Trägermaterialien eignen sich insbesondere Verbindungen der folgendn allgemeinen Formeln

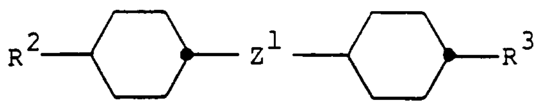

III

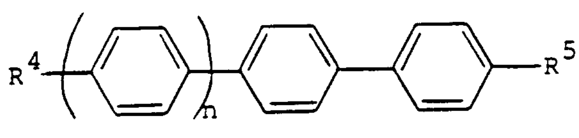

IV

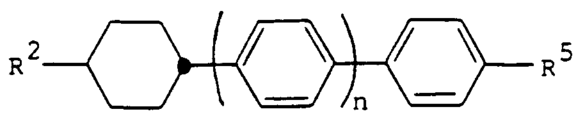

V

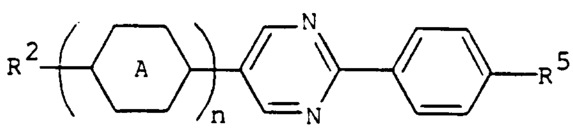

VI

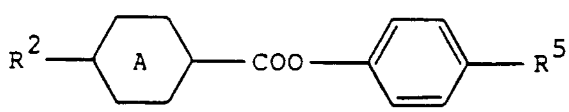

VII

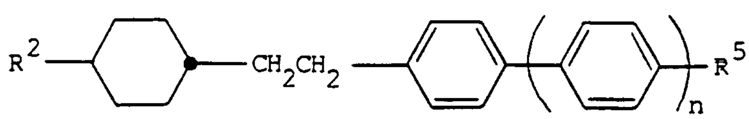

VIII

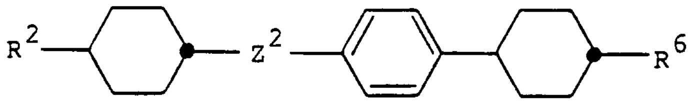

IX

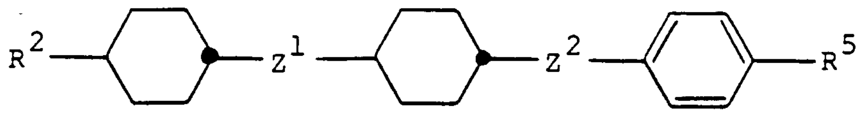

X

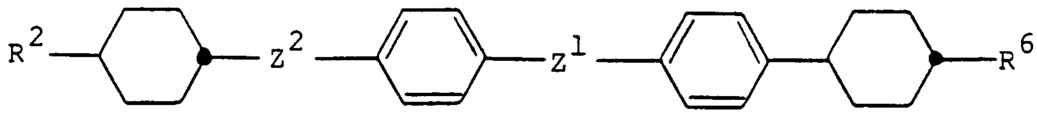

XI

worin $R^2$ und $R^6$ unabhängig voneinander Alkyl oder Alkenyl bezeichnen; $R^3$ Cyano, Alkyl, Alkoxy, Alkenyl, Alkenyloxy, Alkoxymethyl oder Alkenyloxymethyl bedeutet; $Z^1$ eine einfache Kovalenzbindung oder $-CH_2CH_2-$darstellt;n für die Zahl 0 oder 1 steht; $R^4$ Alkyl, Alkoxy, Alkenyl oder Alkenyloxy bezeichnet; $R^5$ Cyano, Alkyl, Alkoxy, Alkenyl oder Alkenyloxy bedeutet; Ring A 1,4-Phenylen oder trans-1,4-Cyclohexylen darstellt; und $Z^2$ eine einfache Kovalenzbindung, -COO- oder $-CH_2CH_2-$ bezeichnet.

Besonders bevorzugt sind daher flüssigkristalline Gemische, welche eine oder mehrere optisch aktive Verbindungen der Formel I und eine oder mehrere Verbindungen aus der Gruppe der Verbindungen der Formeln III-XI enthalten.

$R^2$, $R^3$, $R^4$, $R^5$ und $R^6$ besitzen vorzugsweise höchstens je 12 Kohlenstoffatome, besonders bevorzugt höchstens je 7 Kohlenstoffatome. Bevorzugte Alkenylgruppen sind 1E-Alkenyl, 3E-Alkenyl und 4Z-Alkenyl. Bevorzugte Alkenyloxygruppen sind 2E-Alkenyloxy und 3Z-Alkenyloxy.

Die Erfindung wird durch die folgenden Beispiele weiter veranschaulicht. Im Zusammenhang mit Flüssigkristallphasen und Phasenübergängen bedeuten C eine kristalline Phase, N eine nematische Phase, N* eine cholesterische (chiral nematische) Phase und I die isotrope Phase. Die Helixganghöhe wird mit p bezeichnet und die Wellenlänge des selektiv reflektierten, zirkular polarisierten Lichts mit $\lambda_{max}$. Optische Antipoden besitzen jeweils "spiegelbildliche Eigenschaften", d.h. gleiche Schmelzpunkte etc., führen aber zu entgegengesetztem Helixdrehsinn und entgegengesetzter Zirkularpolarisation des reflektierten Lichts.

Beispiel 1

Ein Gemisch von 0,4 g Naphthalin-2,6-dicarbonsäurechlorid, 0,5 g S(+)-Milchsäure-butylester, 1 ml absolutem Pyridin und 50 ml absolutem Toluol wurde über Nacht unter leichtem Rückfluss und einer Stickstoffatmosphäre erwärmt. Danach wurde das Reaktionsgemisch nacheinander mit 500 ml Wasser, 100 ml verdünnter Salzsäure und nochmals mit 500 ml Wasser gewaschen, über Magnesiumsulfat getrocknet, filtriert und anschliessend eingeengt. Der Rückstand wurde an Kieselgel mit Toluol/Aethylacetat (Vol. 4:1) chromatographisch gereinigt. Umkristallisation aus Aethanol ergab 0,4 g Bis-[(S)-1-(butyloxycarbonyl)äthyl]-2,6-naphthalindicarboxylat mit Smp. (C-I) 81 °C.

Das als Ausgangsmaterial verwendete Naphthalin-2,6-dicarbonsäurechlorid wurde wie folgt hergestellt:

Ein Gemisch aus 1 g Naphthalin-2,6-dicarbonsäure und 100 ml Toluol wurde unter einer Stickstoffatmosphäre mit 2 ml Thionylchlorid versetzt und anschliessend über Nacht unter leichtem Rückfluss erwärmt. Das Reaktionsgemisch wurde eingeengt, dann mit 50 ml absolutem Toluol versetzt und nochmals eingeengt. Diese Prozedur wurde noch zweimal wiederholt. Anschliessend wurde der feste Rückstand in einem Kugelrohr sublimiert. Dies ergab 0,8 g Naphthalin-2,6-dicarbonsäurechlorid.

In analoger Weise können folgende Verbindungen hergestellt werden:

Bis-[(S)-1-(methoxycarbonyl)äthyl]-2,6-naphthalindicarboxylat, Smp. (C-I) 120 °C;
Bis-[(S)-1-(äthoxycarbonyl)äthyl]-2,6-naphthalindicarboxylat, Smp. (C-I) 74 °C;
Bis-[(S)-1-(propyloxycarbonyl)äthyl]-2,6-naphthalindicarboxylat,Smp. (C-I) 121 °C;
Bis-[(S)-1-(pentyloxycarbonyl)äthyl]-2,6-naphthalindicarboxylat;
Bis-[(S)-1-(hexyloxycarbonyl)äthyl]-2,6-naphthalindicarboxylat;
Bis-[(S)-1-(2E-hexenyloxycarbonyl)äthyl]-2,6-naphthalindicarboxylat, Smp. (C-I) 70 °C;
Bis-[(R)-2-pentyl]-2,6-naphthalindicarboxylat, Smp. (C-I) 76 °C;
Bis-[(R)-2-hexyl]-2,6-naphthalindicarboxylat, Smp. (C-I) 53 °C;
Bis-[(R)-2-heptyl]-2,6-naphthalindicarboxylat;
Bis-[(R)-2-octyl]-2,6-naphthalindicarboxylat, Smp. (C-I) 4 °C;
Bis-[(R)-2-nonyl]-2,6-naphthalindicarboxylat.

Beispiel 2

Zur Messung der induzierten Ganghöhe (Pitch) und deren Temperaturabhängigkeit in Flüssigkristallmaterialien wurde die folgende Flüssigkristall-Grundmischung BM-1 verwendet:

| | |
|---|---|
| 5,36 Gew.% | 4'-Aethyl-4-cyanobiphenyl, |
| 3,18 " | 4'-Propyl-4-cyanobiphenyl, |
| 6,08 " | 4'-Butyl-4-cyanobiphenyl, |
| 6,53 " | 4-(trans-4-Propylcyclohexyl)benzonitril, |
| 14,67 " | 4-(trans-4-Pentylcyclohexyl)benzonitril, |
| 5,21 " | 4-Aethyl-1-(trans-4-propylcyclohexyl)benzol, |
| 16,54 Gew.% | 4-Aethoxy-1-[2-(trans-4-propylcyclohexyl)-äthyl]benzol, |
| 5,60 " | 4"-Pentyl-4-cyano-p-terphenyl, |
| 5,71 " | 4'-(trans-4-Pentylcyclohexyl)-4-cyanobiphenyl, |
| 15,95 " | 1-[2-(trans-4-Butylcyclohexyl)äthyl]-4-(trans-4-pentylcyclohexyl)benzol, |
| 4,74 " | 4-[2-(trans-4-Butylcyclohexyl)äthyl]-4'-(trans-4-pentylcyclohexyl)biphenyl, |
| 7,59 " | 4-[2-(trans-4-Butylcyclohexyl)äthyl]-4'-(trans-4-pentylcyclohexyl)-1,1'-äthylendibenzol, |
| 2,84 " | trans-4-[2-(trans-4-Propylcyclohexyl)äthyl]-cyclohexancarbonsäure-4-cyanophenylester; |

Smp. <-30 °C, Klp. (N-I) 90 °C; $\Delta\epsilon$ = 8,5, $\Delta n$ = 0,139 und $\eta$ = 22 mPa•s gemessen bei 22 °C.

Das Verdrillungsvermögen des optisch aktiven Dotierstoffes und dessen Temperaturabhängigkeit wird charakterisiert durch die Parameter A, B und C entsprechend der Reihenentwicklung.

$$\frac{1}{pc} = A + BT_1 + CT_1^2$$

worin und p, c und $T_1$ die folgenden Bedeutungen haben:

$T_1$ = T-22 °C

T = Temperatur in °C

p = Ganghöhe in $\mu$m (ein positiver Wert bedeutet rechtsdrehende Helixstruktur und ein negativer Wert bedeutet linksdrehende Helixstruktur)

c = Konzentration des optisch aktiven Dotierstoffes in Gew.-%.

Mischung M-1

99 Gew.-% BM-1,
1 Gew.-% Bis-[(S)-1-(butyloxycarbonyl)äthyl]-2,6-naphthalindicarboxylat;
Klp. (N*-I) 86,7 °C; p (22 °C) = -5,4 $\mu$m;

A = -0,1863 • $\mu m^{-1}$ • Gew.-%$^{-1}$,
B = 3,605 • $10^{-4}$ • $\mu m^{-1}$ • Gew.-%$^{-1}$ • °$C^{-1}$,
C = 2,838 • $10^{-6}$ • $\mu m^{-1}$ • Gew.-%$^{-1}$ • °$C^{-2}$.

Mischung M-2

99 Gew.-% BM-1,
1 Gew.-% Bis-[(S)-1-(äthoxycarbonyl)äthyl]-2,6-naphthalindicarboxylat;
Klp. (N*-I) 90 °C; p (22 °C) = -4,7 µm;
A = -0,2133 • $\mu m^{-1}$ • Gew.-%$^{-1}$,
B = 4,503 • $10^{-4}$ • $\mu m^{-1}$ • Gew.-%$^{-1}$ • °$C^{-1}$,
C = 2,416 • $10^{-6}$ • $\mu m^{-1}$ • Gew.-%$^{-1}$ • °$C^{-2}$.

Mischung M-3

99 Gew.-% BM-1,
1 Gew.-% Bis-[(R)-2-pentyl]-2,6-naphthalindicarboxylat;
Klp. (N*-I) 89,7 °C; p (22 °C) = +24,8 µm;
A = 0,04039 • $\mu m^{-1}$ • Gew.-%$^{-1}$,
B = -0,341 • $10^{-4}$ • $\mu m^{-1}$ • Gew.-%$^{-1}$ • °$C^{-1}$,
C = -1,156 • $10^{-6}$ • $\mu m^{-1}$ • Gew.-%$^{-1}$ • °$C^{-2}$.

Mischung M-4

99 Gew.-% BM-1,
1 Gew.-% Bis-[(R)-2-hexyl]-2,6-naphthalindicarboxylat;
Klp. (N*-I) 89,8 °C; p (22 °C) = +11,7 µm;
A = 0,0853 • $\mu m^{-1}$ • Gew.-%$^{-1}$,
B = -2,711 • $10^{-4}$ • $\mu m^{-1}$ • Gew.-%$^{-1}$ • °$C^{-1}$,
C = -1,710 • $10^{-6}$ • $\mu m^{-1}$ • Gew.-%$^{-1}$ • °$C^{-2}$.

Mischung M-5

99 Gew.-% BM-1,
1 Gew.-% Bis-[(R)-2-octyl]-2,6-naphthalindicarboxylat;
Klp. (N*-I) 88,6 °C; p (22 °C) = +9,4 µm;
A = 0,1059 • $\mu m^{-1}$ • Gew.-%$^{-1}$,
B = -4,245 • $10^{-4}$ • $\mu m^{-1}$ • Gew.-%$^{-1}$ • °$C^{-1}$,
C = -3,163 • $10^{-6}$ • $\mu m^{-1}$ • Gew.-%$^{-1}$ • °$C^{-2}$.

## Patentansprüche

1. Optisch aktive Naphthalin-2,6-dicarbonsäureester der allgemeinen Formel

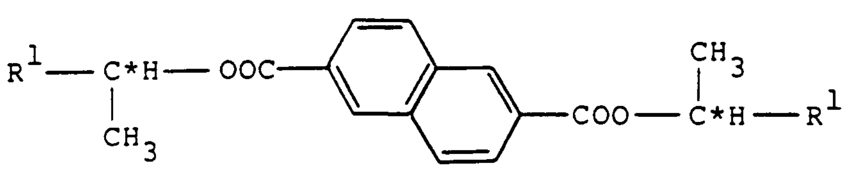

I

worin C* ein chirales Kohlenstoffatom bezeichnet und $R^1$ $C_2$-$C_{12}$-Alkyl, $C_2$-$C_{12}$-Alkenyl, $C_2$-$C_{12}$-Alkoxycarbonyl oder $C_3$-$C_{12}$-Alkenyloxycarbonyl bedeutet.

2. Verbindungen nach Anspruch 1, dadurch gekennzeichnet, dass $R^1$ einen geradkettigen Rest bedeutet.

3. Verbindungen nach Anspruch 1 oder 2, dadurch gekennzeichnet, dass $R^1$ einen Rest mit höchstens 7 Kohlenstoffatomen bedeutet.

4. Flüssigkristallines Gemisch enthaltend ein flüssigkristallines Trägermaterial und eine oder mehrere optisch aktive Verbindungen der in Anspruch 1 definierten Formel I.

5. Flüssigkristallines Gemisch nach Anspruch 4, dadurch gekennzeichnet, dass der Anteil an optisch aktiven Verbindungen der Formel I 0,1-30 Gew.-% beträgt.

6. Flüssigkristallines Gemisch nach Anspruch 4 oder 5, dadurch gekennzeichnet, dass es eine oder mehrere optisch aktive Verbindungen der Formel I und eine oder mehrere Verbindungen enthält aus der Gruppe der Verbindungen der allgemeinen Formeln

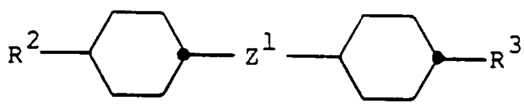

III

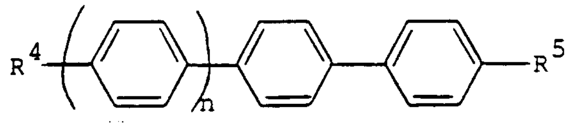

IV

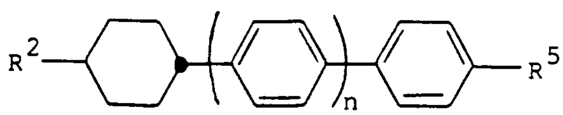

V

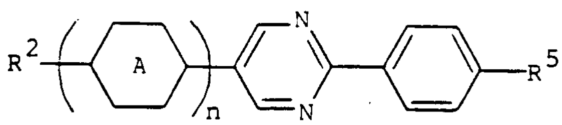

VI

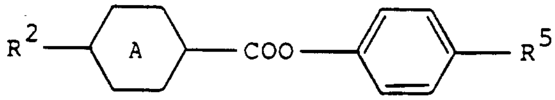

VII

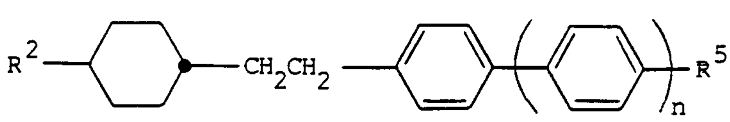

VIII

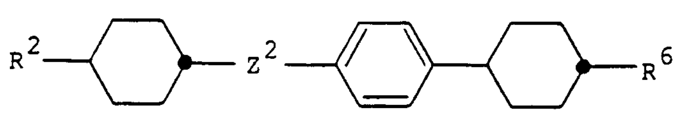

IX

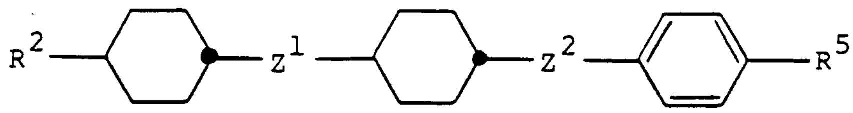

X

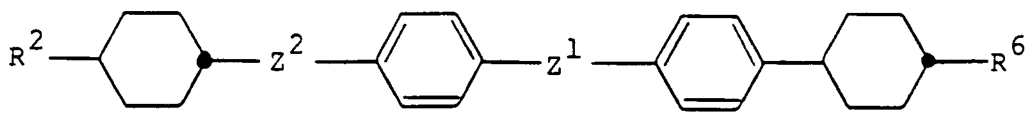

XI

worin $R^2$ und $R^6$ unabhängig voneinander Alkyl oder Alkenyl bezeichnen; $R^3$ Cyano, Alkyl, Alkoxy, Alkenyl, Alkenyloxy, Alkoxymethyl oder Alkenyloxymethyl bedeutet; $Z^1$ eine einfache Kovalenzbindung oder $-CH_2CH_2$-darstellt;n für die Zahl 0 oder 1 steht; $R^4$ Alkyl, Alkoxy, Alkenyl oder Alkenyloxy bezeichnet; $R^5$ Cyano, Alkyl, Alkoxy, Alkenyl oder Alkenyloxy bedeutet; Ring A 1,4-Phenylen oder trans-1,4-Cyclohexylen darstellt; und $Z^2$ eine einfache Kovalenzbindung, -COO- oder $-CH_2CH_2-$ bezeichnet.

**7.** Verfahren zur Herstellung der optisch aktiven Naphthalin-2,6-dicarbonsäureester der in Anspruch 1 definierten Formel I, dadurch gekennzeichnet, dass man Naphthalin-2,6-dicarbonsäure oder ein geeignetes Derivat der Naphthalin-2,6-dicarbonsäure mit einer optisch aktiven Verbindung der allgemeinen Formel

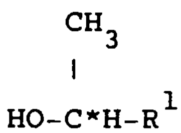

II

worin C* und $R^1$ die in Anspruch 1 gegebenen Bedeutungen haben,
verestert.

**8.** Verwendung flüssigkristalliner Gemische gemäss Anspruch 4 für optische oder elektro-optische Zwekke.

**Claims**

**1.** Optically active naphthalene-2,6-dicarboxylic acid esters of the general formula

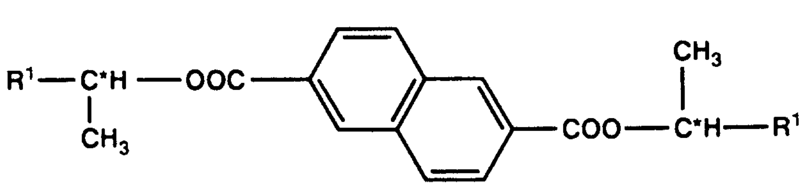

I

wherein C* denotes a chiral carbon atom and $R^1$ signifies $C_2$-$C_{12}$-alkyl, $C_2$-$C_{12}$-alkenyl, $C_2$-$C_{12}$-alkoxycarbonyl or $C_3$-$C_{12}$-alkenyloxycarbonyl.

**2.** Compounds according to claim 1, characterized in that $R^1$ signifies a straight-chain residue.

**3.** Compounds according to claim 1 or 2, characterized in that $R^1$ signifies a residue having a maximum of 7 carbon atoms.

4. A liquid crystalline mixture containing a liquid crystalline carrier material and one or more optically active compounds of formula I defined in claim 1.

5. A liquid crystalline mixture according to claim 4, characterized in that the content of optically active compounds of formula I is 0.1-30 wt.%.

6. A liquid crystalline mixture according to claim 4 or 5, characterized in that it contains one or more optically active compounds of formula I and one or more compounds from the group of compounds of the general formulae

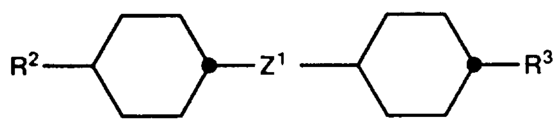 III

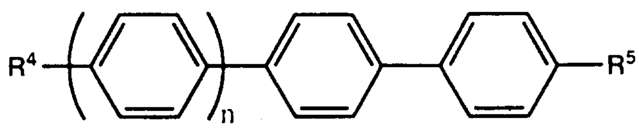 IV

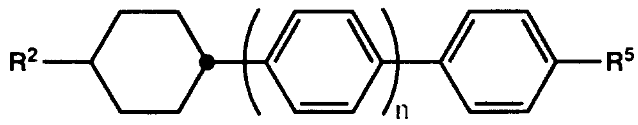

V

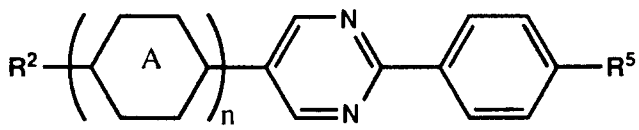

VI

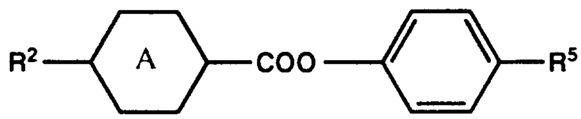

VII

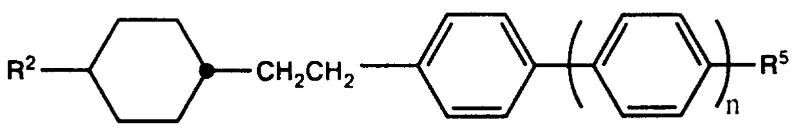

VIII

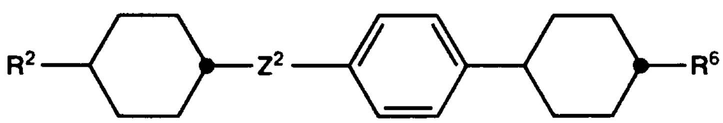

IX

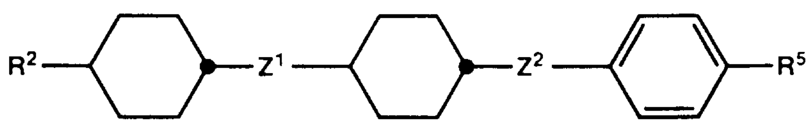

X

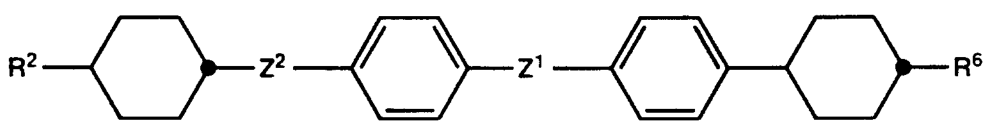

XI

wherein $R^2$ and $R^6$ each independently denote alkyl or alkenyl; $R^3$ signifies cyano, alkyl, alkoxy, alkenyl, alkenyloxy, alkoxymethyl or alkenyloxymethyl; $Z^1$ represents a single covalent bond or $-CH_2CH_2-$; n stands for the number 0 or 1; $R^4$ denotes alkyl, alkoxy, alkenyl or alkenyloxy; $R^5$ signifies cyano, alkyl, alkoxy, alkenyl or alkenyloxy; ring A represents 1,4-phenylene or trans-1,4-cyclohexylene; and $Z^2$ denotes a single covalent bond, -COO- or $-CH_2CH_2-$.

**7.** A process for the manufacture of the optically active naphthalene-2,6-dicarboxylic acid esters of formula I defined in claim 1, characterized by esterifying naphthalene-2,6-dicarboxylic acid or a suitable derivative of naphthalene-2,6-dicarboxylic acid with an optically active compound of the general formula

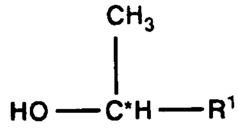

II

wherein C* and $R^1$ have the significances given in claim 1.

**8.** The use of liquid crystalline mixtures in accordance with claim 4 for optical or electro-optical purposes.

## Revendications

1. Esters naphtalène-2,6-dicarboxyliques possédant l'activité optique, qui répondent à la formule générale

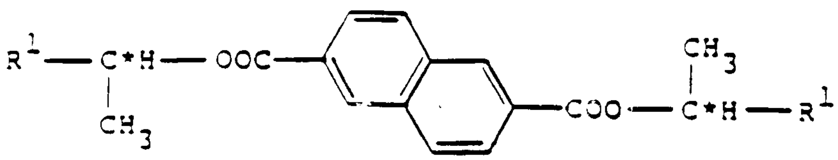

I

dans laquelle C* représente un atome de carbone chiral et $R^1$ un groupe alkyle en C2-C12, alcényle en C2-C12, alcoxycarbonyle en C2-C12 ou alcényloxycarbonyle en C3-C12.

2. Composés selon revendication 1, caractérisés en ce que $R^1$ représente un groupe à chaîne droite.

3. Composés selon revendication 1 ou 2, caractérisés en ce que $R^1$ représente un groupe contenant au maximum 7 atomes de carbone.

4. Mélange à cristaux liquides contenant un véhicule à cristaux liquides et un ou plusieurs composés de formule I de la revendication 1, possédant l'activité optique.

5. Mélange à cristaux liquides selon revendication 4, caractérisé en ce que la proportion des composés de formule I possédant l'activité optique est de 0,1 à 30 % en poids.

6. Mélange à cristaux liquides selon revendication 4 ou 5, caractérisé en ce qu'il contient un ou plusieurs composés de formule I possédant l'activité optique et un ou plusieurs composés choisis parmi ceux qui répondent aux formules générales

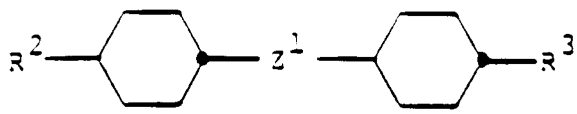

III

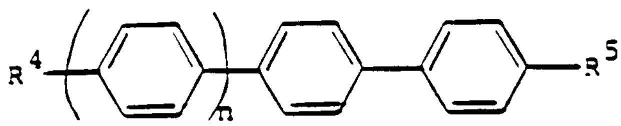

IV

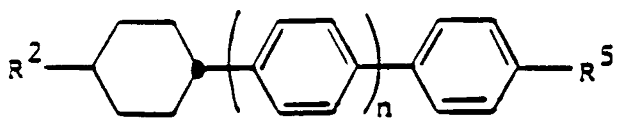

V

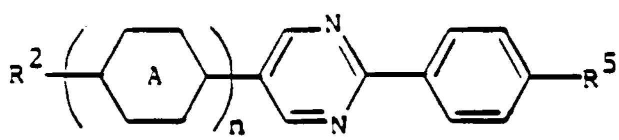

VI

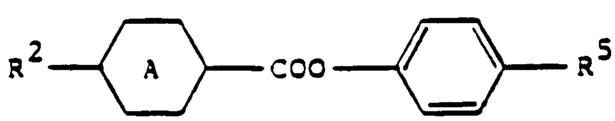

VII

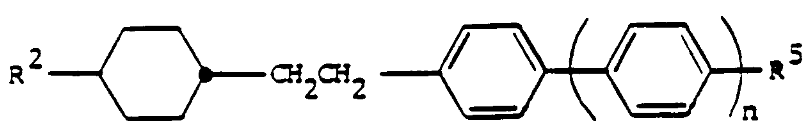

VIII

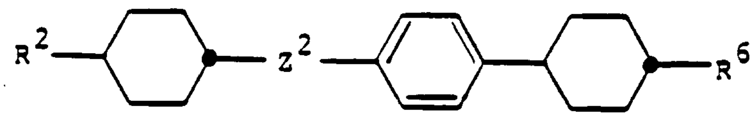

IX

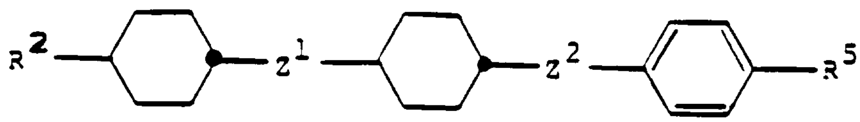

X

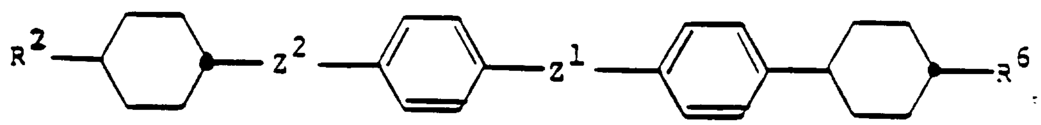

XI

dans lesquelles $R^2$ et $R^6$ représentent chacun, indépendamment l'un de l'autre, un groupe alkyle ou alcényle ;
$R^3$ représente un groupe cyano, alkyle, alcoxy, alcényle, alcényloxy, alcoxyméthyle ou alcényloxyméthyle ;
$Z^1$ représente une liaison covalente simple ou un groupe -CH2CH2-; n est égal à 0 ou 1 ;
$R^4$ représente un groupe alkyle, alcoxy, alcényle ou alcényloxy ;
$R^5$ représente un groupe cyano, alkyle, alcoxy, alcényle ou alcényloxy ;
le cycle A est un cycle 1,4-phénylène ou trans-1,4-cyclohexylène ; et
$Z^2$ représente une liaison covalente simple, -COO- ou-CH2CH2-.

**7.** Procédé de préparation des esters naphtalène-2,6-dicarboxyliques de formule I de la revendication 1 possédant l'activité optique, caractérisé en ce que l'on estérifie l'acide naphtalène-2,6-dicarboxylique ou un dérivé approprié de cet acide par un composé, possédant l'activité optique et répondant à la formule générale

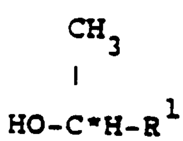

II

dans laquelle C* et $R^1$ ont les significations indiquées dans la revendication 1.

**8.** Utilisation de mélanges à cristaux liquides selon revendication 4 dans des applications optiques ou électrooptiques.